(19) Europäisches Patentamt

European Patent Office

Office européen des brevets

(11) Publication number: **0 272 578 B1**

# EUROPEAN PATENT SPECIFICATION

(45) Date of publication of patent specification: **24.03.93**

(51) Int. Cl.⁵: **C12Q 1/60**, C12Q 1/28, C12Q 1/44

(21) Application number: **87118510.4**

(22) Date of filing: **14.12.87**

(54) **Multilayer analysis elements for the determination of total cholesterol.**

(30) Priority: **18.12.86 JP 302325/86**

(43) Date of publication of application:
**29.06.88 Bulletin 88/26**

(45) Publication of the grant of the patent:
**24.03.93 Bulletin 93/12**

(84) Designated Contracting States:
**DE GB**

(56) References cited:
**EP-A- 0 091 026
EP-A- 0 176 357
EP-A- 0 244 825
US-A- 3 983 005
US-A- 4 275 152**

**RESEARCH DISCLOSURE, vol. 177, no. 126, October 19741; "Multilayer analytical elements for use in the assay of cholesterol", pp. 51-56***

**CLINICAL CHEMISTRY, vol. 25, no. 1, January 1979, Easton, PA (US); J.ABELE et al., pp. 132-135***

(73) Proprietor: **Fuji Photo Film Co., Ltd.
210 Nakanuma Minamiashigara-shi
Kanagawa-ken(JP)**

(72) Inventor: **Arai, Fuminori, c/o Fuji Photo Film
Co. Ltd.
No. 11-46, Senzui 3-chome
Asake-shi, Saitama(JP)**
Inventor: **Igarashi, Takeshi, c/o Fuji Photo
Film Co. Ltd.
No. 11-46, Senzui 3-chome
Asake-shi, Saitama(JP)**

(74) Representative: **Kraus, Walter, Dr. et al
Patentanwälte Kraus, Weisert & Partner
Thomas-Wimmer-Ring 15
W-8000 München 22 (DE)**

## Description

This invention relates to an improvement in a dry type multilayer analysis element for quantitatively analyzing total cholesterol contained in aqueous samples, such as body fluids (e.g., blood), foods and drinks.

## BACKGROUND OF THE INVENTION

In the quantitative analysis of cholesterol and cholesterol esters which are present in the form of lipoproteins in body fluids such as blood, by using a dry type multilayer analysis element, blood, as an aqueous sample, is used without being diluted with water, physiological saline, a pH buffer, etc. Hence, the color reaction in the analysis element does not proceed rapidly. Further, substances in the body fluid (e.g., neutral fats in the blood), make accurate analysis difficult.

In an attempt to solve the above problem, Japanese Patent Application (OPI) Nos. 96378/78 and 85200/86 (corresponding English literatures are shown hereinafter) and CLINICAL CHEMISTRY, 28, 1159-1162 (1982) proposed the joint use of an alkyl phenoxy polyethoxy ethanol. (The term "OPI" indicates an unexamined published patent open to public inspection.) However, techniques proposed in these references have been found to have the following problems:

(1) Cholesterol esterase activity is inhibited.

(2) During storage before use, the dry type multilayer analysis element has a strong tendency to absorb water. Hence, the enzyme activity in the element is decreased or lost, and the performance of the element is degraded.

CLINICAL CHEMISTRY, 20, 470-475 (1974) and Japanese Patent Application (OPI) No. 125796/75 describe that cholesterol esters are efficiently hydrolyzed in aqueous solution by using cholesterol esterase and bile acid or its salt in combination. When this technique is applied to the dry type multilayer analysis element, spreading of an aqueous sample, particularly blood (whole blood, plasma or serum) is poor on a porous spreading layer, and the accuracy of analysis decreases drastically.

## SUMMARY OF THE INVENTION

Accordingly an object of the present invention is to provide an excellent dry type multilayer analysis element for total cholesterol determination which offers a solution to the aforesaid problems (such as the inhibition of cholesterol esterase activity, the degraded performance during storage, and the low accuracy of analysis).

In order to solve the above-described problems, it has been found that by using a cholesterol analysis reagent composition comprising a combination of bile acid or its salt with an alkyl phenoxy polyethoxy ethanol containing a polyoxyethylene chain with at least 16 (and usually at least 20 on an average) oxyethylene units, a dry type multilayer analysis element can be obtained where inhibitions of the activity of an enzyme having cholesterol esterase activity is decreased and which has increased storage stability.

It has also been found that when a liquid sample containing various substances which interfere with the coloration of the color reagent composition, such as blood (whole blood, plasma, or serum), is used, the color reaction proceeds smoothly if carried out in the presence of bile acid or its salt and an alkyl phenoxy polyethoxy ethanol containing a polyoxyethylene chain with at least 16 (and generally at least 20 on an average) oxyethylene units, and that as a result, a high optical density of the coloration can be obtained which permits accurate colorimetry of the total cholesterol content of the sample.

According to this invention, there is provided an improved dry type multilayer analysis element for determination of total cholesterol comprising a light-transmissive water-impermeable support, at least one hydrophilic polymer layer (to be referred to as a hydrophilic layer) on said support and a spreading layer on said hydrophilic polymer layer(s) and further containing a cholesterol analysis reagent composition comprising at least one enzyme having cholesterol esterase activity, cholesterol oxidase, peroxidase and a color reagent composition in at least one of said spreading layer and the hydrophilic polymer layer(s), wherein said element further includes a bile acid compound (at least one of bile acids, bile acid derivatives and salts thereof) and an alkyl phenoxy polyethoxy ethanol containing polyoxyethylene chains with at least 16 (and generally at least 20 on an average) oxyethylene units are included in at least one of the spreading layer and the hydrophilic layer(s).

2

DETAILED DESCRIPTION OF THE INVENTION

The element of the present invention usually forms an integral multilayer analysis element.

The light-transmissive water-impermeable support used in this invention may be any of those used generally in multilayer analysis elements. Specifically, there may be used, for example, a transparent support having a thickness of 50 $\mu$m to 1 mm, preferably 80 $\mu$m to 300 $\mu$m , made of a polymer such as polyethylene terephthalate, bisphenol A, polycarbonate, polystyrene, and cellulose esters (e.g., cellulose diacetate, cellulose triacetate and cellulose acetate propionate). As required, the adhesion of the support to the hydrophilic layer and other layers to be provided on the support can be strengthened by providing a subbing layer or an adhesive layer known from the specifications of the above-cited patent documents.

A hydrophilic layer can be allowed to function as a water-absorbing layer which does not contain a reagent component, swells upon water absorption, and optionally may receive a species to be detected (a dye formed) and accumulate it, and as a detecting layer which contains a mordant having a negative or a positive charge and being able to receive and fix the species to be detected and swells upon water absorption. This layer may contain a pH buffer composition which stabilizes the pH at the time of the color reaction. It may be formed of a hydrophilic polymer binder alone or with another component containing therein. The hydrophilic polymer binder is a natural or synthetic hydrophilic polymer having a swelling ratio at the time of water absorption of 150% to 2000%, preferably 250% to 1500%, at 30°C.

Examples of the hydrophilic polymer binder include gelatins (acid-processed gelatin, deionized gelatin, etc.), gelatin derivatives (such as phthalated gelatin, and hydroxyacrylate grafted gelatin), agarose, pullulan, pullulan derivatives, polyacrylamide, polyvinyl alcohol, and polyvinyl pyrrolidone, as disclosed, for example, in Japanese Patent Application (OPI) Nos. 171864/84 and 108753/85.

The hydrophilic layer generally has a thickness in the dry state of 1 $\mu$m to 100 $\mu$m , preferably 3 $\mu$m to 50 $\mu$m , especially preferably 5 $\mu$m to 30 $\mu$m , and is preferably substantially transparent.

The hydrophilic layer may include some or all of a pH buffer, enzymes, a color reagent composition and an optional reagent (such as a ferrocyamide which is used to increase a coloring speed and increase a coloring efficiency), and also one or more of known mordants, basic polymers (acts as a basic buffer or a base), acidic polymers (acts as an acidic polymer or an acid), etc. In this case, the hydrophilic layer functions as a reagent layer or a coloring reagent layer.

Examples of the spreading layers include non-fibrous isotropic porous developing layers such as a porous layer containing continuous open microvoids in which a membrane filter (a blushed polymer layer), and polymeric microbeads, glass microbeads and diatomaceous earth held in a hydrophilic polymer binder disclosed, for example, in Japanese Patent Publication No. 21677/78, and a porous layer containing continuous open microvoids (a three-dimensional latice particle structure layer) in which polymeric microbeads and glass microbeads are bonded in point-to-point contact with a polymer adhesive not swellable with water as disclosed in Japanese Patent Application (OPI) No. 90859/80; fibrous porous layers such as the spreading layers of woven fabrics disclosed, for example, in Japanese Patent Application (OPI) Nos. 164356/80 and 66359/82; the spreading layer of knitted fabric disclosed in Japanese Patent Application (OPI) No. 222769/85, and spreading layer composed of paper containing organic polymer fiber pulp disclosed in Japanese Patent Application (OPI) No. 148250/82.

The spreading layer is superimposed on the hydrophilic layer either directly or as required, through an adhesive layer. The spreading layer may include a part or all of the enzymes, the color reagent composition and as required, a ferrocyanide and other reagents.

Of the enzymes, one having cholesterol esterase activity is preferably included in the porous spreading layer.

In the multilayer analysis element of this invention, there may be incorporated reagent layers, light-shielding layers, light-reflecting layers, filtration layers, semipermeable layers, barrier layers and diffusion preventing layers (migration preventing layers) as disclosed in the above-cited patent documents, and a layer having two or more functions possesed by single layer of these layers.

The reagent layers contains a part or all of the enzymes, the color reagent composition and as required, the ferrocyanide and other reagents. It is a non-porous water absorbing and preferably swellable or a microporous water-permeable layer. It is a preferable embodiment that a water-absorbing layer is provided on a support and a reagent layer is provided thereon.

A hydrophilic polymer binder used in the substantially non-porous water-absorbing reagent layer acts as a medium for dissolving or dispersing the reagent substantially uniformly and at least has an action of absorbing water from the liquid analysis sample and transporting the component to be analyzed to the reagent layer together with water. The hydrophilic polymer binder may be any one of the water-absorbing hydrophilic polymer binders used in the aforesaid hydrophilic layer. The substantially non-porous reagent layer has a thickness in the dry state generally of 3 $\mu$m to 50 $\mu$m , preferably 5 $\mu$m to 30 $\mu$m , and the reagent layer is preferably substantially transparent.

The microporous water-permeable reagent layer is a layer in which a reagent or a reagent compositions is included in a microporous structure layer consisting of solid fine particles and a hydrophilic polymer binder therefor. The microporous structure layer, as referred to herein, is a layer of a structure composed of microporous fine particles of non-porous fine particles and a hydrophilic polymer binder bonding the fine particles to one another to retain a microporous open void structure.

Examples of the microporous fine particles or the non porous fine particles used in the porous reagent layer are cellulose particles such as fine powders or fine particles of cellulose or microcrystalline cellulose, fine particles of silicon dioxide compounds, such as silica, and diatomaceous earth, fine particles of silicates, such as zeolite, polymer microbeads, glass microbeads, and various ceramic beads. The hydrophilic polymer binder may be properly selected from the same polymers as the water-absorbing hydrophilic polymers used in the above-described hydrophilic layer, and the aqueous latices of copolymers containing about 2% or more of hydrophilic recurring units as disclosed in Japanese Patent Application (OPI) No. 145965/84 may also be used. The microporous reagent layer has a thickness in the dry state generally of 7 $\mu$m to 50 $\mu$m , preferably 10 $\mu$m to 30 $\mu$m.

The reagent layer may include known pH buffer compositions, polymer pH buffers, basic polymers, acid polymers, polymeric mordants, etc.

In one preferred embodiment, a water-absorbing layer is disposed between the reagent layer and the support. In an especially preferred embodiment, the color reagent composition is included in the hydrophilic layer, and the enzyme, the bile acid compound and the alkyl phenoxy polyethoxy ethanol are included in the porous spreading layer.

A light-shielding layer may be provided on the reagent layer or any other hydrophilic layer. The light-shielding layer is a water-permeable or water-penetratable layer composed of fine particles or powders (to be simply referred to as fine particles) having a light-shielding property or both light-shielding and light-reflting properties dispersed and retained in a small amount of a film-forming hydrophilic polymer binder. The light-shielding layer shields the color of the aqueous liquid sample, particularly, the red color of hemoglobin contained in a whole blood sample, spotted and fed onto the spreading layer when the color formed in the reagent layer or the hydrophilic layer is reflted and measured from the side of the light-transmissive support. It also acts as a light reflting layer or a background layer.

Examples of the fine particles having both light-shielding and light-reflting properties are fine particles of titanium dioxide (fine crystalline particles having a particle diameter of from 0.1 $\mu$m to 1.2 $\mu$m of rutile, anatase or burkeite type titanium dioxide), fine particles of barium sulfate, and fine particles or fine flakes of aluminum. Among these, fine particles of titanium dioxide and fine particles of barium sulfate are preferred.

Examples of the light-shielding fine particles are carbon black, gas black and carbon microbeads.

The film-forming hydrophilic polymer binder may be the same hydrophilic polymers as used in the hydrophilic layer, and also weakly hydrophilic regenerated cellulose and cellulose acetate. Preferred polymer binders are gelatin, gelatin derivatives and polyacrylamide. The gelatin and gelatin derivatives may be used as a mixture with known hardeners (crosslinking agents).

The light-shielding layer may by provided by coating an aqueous dispersion of light-shielding fine particles and a hydrophilic polymer on the reagent layer or the hydrophilic layer and drying it by a known method. The volume ratio of the hydrophilic polymer binder to 10 parts of the light-shielding fine particles in the dry state is generally from 2.5 to 7.5 preferably from 3.0 to 6.5 parts. When the light-shielding fine particles are those of titanium dioxide, the weight ratio of the polymer binder to 10 parts of fine particles of titanium dioxide is from 0.6 to 1.8, preferably 0.8 to 1.5 parts. The thickness of the light-shielding layer on drying is generally from 3 $\mu$m to 30 $\mu$m , preferably from 5 $\mu$m to 20 $\mu$m.

An adhesive layer may be provided to stack and bond the spreading layer, the light-shielding layer, etc. When a porous spreading layer is provided on the light-shielding layer, it is preferable to provide the adhesive layer. However, when a spreading layer is provided on a hydrophilic polymer-containing layer such as water-absorbing layer, a reagent layer, etc., the adhesive layer may not be provided depending on the type and properties of the hydrophilic polymer. The adhesive layer is composed mainly of a hydrophilic polymer which when wetted with water or swollen with water, can bond the spreading layer to form a integral structure. The hydrophilic polymer used in the adhesive layer may be any of the same hydrophilic polymers used in the above-described hydrophilic layer. Preferred hydrophilic polymers used in the adhesive layer are gelatin, gelatin derivatives, polyvinyl alcohol and polyacrylamide. The adhesive layer has a thickness in the dry state generally of 0.5 $\mu$m to 20 $\mu$m , preferably 1 $\mu$m to 10 $\mu$m. The adhesive layer may contain a surface-active agent, preferably a nonionic surface-active agent such as a nonionic surface-active agent having a chain structure composed of 8 to 15 oxyethylene or oxypropylene groups linked to each other.

The adhesive layer may be provided by coating an aqueous solution containing a hydrophilic polymer and as optional components, the surface-active agent, etc., on the reagent layer, the hydrophilic layer or on the light-shielding layer by a known method.

The cholesterol analysis color reagent composition used in the element of this invention comprises at least one enzyme having cholesterol esterase activity, cholesterol oxidase, peroxidase, a color reagent composition, at least one compound selected from the group consisting of bile acids, bile acid derivatives and salts thereof, and an alkyl phenoxy polyethoxy ethanol containing a polyoxyethylene chain composed of at least 16 (and generally at least 20 on an average) oxyethylene units. The composition may also contain a ferrocyanide.

The cholesterol esterase (Enzyme Code 3.1.1.13; hereinafter "Enzyme Code" is simply referred to as "EC ") is an enzyme which converts cholesterol esters in the analysis sample into cholesterol. Cholesterol oxidase (EC 1.1.3.6) is an enzyme which oxidizes cholesterol to form hydrogen peroxide. Peroxidase (EC 1.11.1.7) is an enzyme which participates in an reaction of oxidizing the color reagent composition by utilizing hydrogen peroxide to form a color. Not only enzymes classified as EC 3.1.1.13 may be used as the cholesterol esterase, but there can also be used other enzymes having cholesterol esterase activity either singly (for example, Lipase M® manufactured by Enzyme Development Corp. and Lipase 3000® manufactured by Wilson Laboratories as described in Japanese Patent Publication No. 45599/1981) or combinations of two or more such enzymes, for example, a combination of lipase having cholesterol esterase activity described in Japanese Patent Publication No. 45599/81 (e.g., Lipase M® and Lipase 3000®) with protease, for example, chymotrypsin (EC 3.4.21.1), papain (EC 3.4.21.2), or bromelain (EC 3.4.22.4), or Pronase®).

Cholesterol oxidase (EC 1.1.3.6) is an enzyme which oxidizes cholesterol to generate hydrogen peroxide. It may be selected properly from commercially available enzymes. As required, it may be used together with a coenzyme such as FAD (flavin adenine dinucleotide).

As the peroxidase, peroxidases (EC 1.11.1.7) derived from a plant or an animals described in Japanese Patent Publications No. 45599/1981 and 5520/1982, and a peroxidase (EC 1.11.1.7) or derived from a microorganism as described in Japanese Patent Publications No. 5035/1983 may be used. Of these, non-specific peroxidases derived from plant or microorganism are preferred. Examples of the preferred peroxidases are peroxidases extracted from horseradish and radish, and peroxidases extracted from microorganisms of the genera Cochliobolus and Curvularia.

Examples of the bile acids are cholic acid, deoxycholic acid, lithocholic acid, and kenodeoxycholic acid. Examples of the bile acid derivatives are taurocholic acid, glycolic acid, taurodeoooxycholic acid, and glycodeoxycholic acid. The salts of the bile acids and bile acid derivatives may, for example, be sodium, potassium and lithium salts of these compounds. In the multilayer analysis element of this invention, at least one compound selected from the above-mentioned bile acids, and bile acid derivatives and their salts (to be generally referred to as the bile acid compounds in this invention) is included in any of the layers. Preferably, the bile acid compound is included in the layer or in a layer adjoining the spreading layer. Especially preferably, the bile acid compound is included in the same layer together with the alkyl phenoxy polyethoxy ethanol and one or more of enzymes having cholesterol esterase activity.

The alkyl groups in the alkyl phenoxy polyethoxy ethanol containing a polyoxyethylene chain composed of at least 16 (and generally at least 20 on an average) oxyethylene units (to be referred to simply as the alkyl phenoxy polyethoxy ethanol; this compound is also called a polyoxyethylene alkylphenyl ether) may be various alkyl groups, preferably linear or branched alkyl groups having from 1 to 20, more preferably from 7 to 10 carbon atoms, and the most preferably having 8 or 9 carbon atoms such as an octyl, 1,1,3,3-tetramethylbutyl or nonyl group. The number of the oxyethylene units in the polyoxyethylene chain is from preferably 16 to about 60, and the average number of the units is preferably from 20 to 50 and more preferably from 30 to 50. The alkyl group may be present at any position of the phenyl group, however, generally, it is at p- or m-position with respect to the oxygen atom of the phenoxy group.

Examples of the alkyl phenoxy polyethoxy ethanol are octyl phenoxy polyethoxy ethanol (a mixture of compounds containing 20 to 40 oxyethylene units on an average), nonyl phenoxy polyethoxy ethanol (containing 20 to 40 oxyethylene units on an average), 1,1,3,3-tetramethyl butyl phenoxy polyethoxy ethanol (containing 20 to 40 oxyethylene units on an average). Preferably, the alkyl phenoxy polyethoxy ethanol is included in the same layer together with one or more enzymes having cholesterol esterase activity and the bile acid compound or in a layer adjacent to the layer containing enzymes and the bile acid compound. Two or more alkyl phenoxy polyethoxy ethanols may be used in combination, or one or more of alkyl pyhenoxy polyethoxy ethanols may be used in combination with another type of surface-active agent, particularly a nonionic surface-active agent.

The color reagent composition is, for example, (1) a composition containing a chromogen and a coupler, and oxidatively couples with the chromogen by hydrogen peroxide in the presence of peroxidase to coloring by forming a quinoneimine dye, or (2) a leuco dye which is oxidized by hydrogen peroxide in the presence of peroxidase to form a dye.

Examples of the chromogen are 4-aminoantipyrine (also called 4-aminophenazone, i.e., 1-phenyl-2,3-dimethyl-4-amino-3-pyrazoline-5-one) described in Ann. Clin. Biochem, 6, 24-27 (1969), trisubstituted-4-amino-3-pyrazoline-5-ones such as 1-(2,4,6-trichlorophenyl)-2,3-dimethyl-4-amino-3-puyrazoline-5-one) and 1-(3,5-dichlorophenyl)-2,3-dimethyl-4-amino-3-pyrazoline-5-one described in Japanese Patent Application (OPI) No. 54962/1984, and 4-aminoantipyrine analogs such as 1-phenyl-2,3-dimethyl-4-dimethylamino-3-pyrazoline-5-one described in Japanese Patent Publication No. 25840/1980. Of these, 4-aminoantipyrine, 1-(2,4,6-trichlorophenyl)-2,3-dimethyl-4-amino-3-pyrazoline-5-one and 1-(3,5-dichlorophenyl)-2,3-dimethyl-4-amino-3-pyrazoline-5-one) are preferred.

Examples of the couplers include the phenols described in Ann, clim. Biochem., 6, 24-27 (1969), Japanese Patent Publications Nos. 25840/1980, 45599/1981 and 18628/1983, Japanese Patent Application (OPI) Nos. 164356/1980, 124398/1981 and 155852/1981; phenolsulfonic acids (including alkali metal salts and alkaline earth metal salts thereof) such as 2-hydroxy-1-benzensulfonic acid, 4-hydroxy-1-benzenesulfonic acid, 3,5-dichloro-2-hydroxy-1-benzenesulfonic acid and 2-hydroxy-3-methoxy-1-benzenesulfonic acid; 1-naphthol and 2-naphthol; dihydroxynaphthalenes such as 1,7-dihydroxynaphthalene; naphtholsulfonic acids (including alkali metal salts and alkaline earth metal salts thereof) such as 1-hydroxy-2-naphthalenesulfonic acid and 1-hydroxy-4-naphthalenesulfonic acid; and other phenol or naphthol derivatives. Of these compounds, 1,7-dihydroxynaphthalene, 1-hydroxy-2-naphthalenesulfonic acid (including its Na, K and Li salts) and 3,5-dichloro-2-hydroxy-1-benzenesulfonic acid (including its Na, K and Li salts).

Examples of the leuco dye are triarylimidazole leuco dyes such as 2-(4-hydroxy-3,5-dimethoxyphenyl)-4,5-bis[4-(dimethylamino)phenyl]imidazole described in Japanese Patent Publication No. 5519/1982; diarylimidazole leuco dyes such as 2-(3,5-dimethoxy-4-hydroxyphenyl)-4-[4-(dimethylamino)phenyl]5-phenethylimidazole described in Japanese Patent Application (OPI) No. 193352/1984; diarylindolylimidazole leuco dyes such as 2-(2-phenyl-3-indolyl)-4,5-di[4-(dimethylamino)phenyl]-imidazole described in Japanese patent Application (OPI) No. 4960/1986; triarylmonoacylimidazole leuco dyes such as 2-(4-hydroxy-3,5-dimethoxyphenyl)-3-acetyl-4,5-bis[4-(diethylamino)phenyl]imidazole described in Japanese Patent Application (OPI) No. 229868/1986; and triarylmonoalkylimidazole leuco dyes such as 2-(4-hydroxy-3,5-dimethoxyphenyl)-3-methyl-4,5-bis[4-(diethylamino)phenyl]imidazole.

A ferrocyanide compound may be added as an optional component to the cholesterol analysis color reagent composition.

A compound which contains or releases a ferrocyanide ion (a hexacyanoferrate (II) ion, i.e., $[Fe(CH)_6]^{-4\ominus}$) may be used as the ferrocyanide compound. Alkali metal or alkaline earth metal hexacyanoferrates (II) or mixture of these metal salts may be used as the ferrocyanide compound. Specific examples of the ferrocyanide compound are sodium ferrocyanide $Na_4[Fe(CN)_6]$, potassium ferrocyanide $K_4[Fe(CN)_6]$, lithium hexacyanoferrate (II), magnesium hexacyanoferrate (II), and calcium hexacyanoferrate (II). Of these, potassium ferrocyanide and sodium ferrocyanide are preferred.

Among the components of the choloesterol analysis color reagent composition, the enzymes, the bile acid compound and the alkyl phenoxy polyethoxy ethanol are preferably included in the porous spreading layer. The color reagent composition and the ferrocyanide as an optional component may be included in one or more of the porous spreading layer and a layer adjoining or not adjoining the porous spreading layer such as an independent reagent layer, a hydrophilic layer and a water-absorbing layer.

The amounts of the components of the cholesterol analysis color reagent composition used in the element of the present invention (the amounts coated per $m^2$ of the element) are as follows:-

At least one enzyme having cholesterol esterase activity: 500 to 50,000 IU, preferably 1,000 to 30,000 IU (as the cholesterol esterase activity value).

Cholesterol oxidase: 1,000 to 30,000 IU, preferably 1,500 to 20,000 IU.

Peroxidase: 1,000 to 100,000, preferably 2,000 to 60,000 IU.

Chromogen or leuco dye: 0.5 to 10 millimoles, preferably 1 to 5 millimoles.

Coupler: 2.5 to 50 millimoles, preferably 5 to 25 millimoles.

Ferrocyanide: 0.1 to 10 millimoles, preferably 0.5 to 5 millimoles.

Bile acid compound: 0.2 g to 10.0 g, preferably 0.5 to 5.0 g.

Alkyl phenoxy polyethoxy ethanol containing a polyoxyethylene chain composed of at least 16 (and generally at least 20 on an average) oxyethylene units: 0.5 g to 12.0 g, preferably 0.8 g to 8.0 g.

A known buffer capable of buffering the pH values at the time of conducting analysis of a liquid sample to a desired value in the range of 5.5 to 9.0, preferably 7.7 to 8.5, may be included in the multilayer

6

analysis element of the present invention.

Buffers that can be used are, for example, the pH buffer systems described in "Manual of Chemistry, Fundamentals" edited by the Japanese Chemical Society (published in 1966 by Maruzen Co., Ltd., Tokyo), pages 1312-1320; "Data for Biochemical Research" edited by R. M. C. Dawson et al., 2nd edition (published by Oxford at the Clarendon Press, 1969), pages 476-508, Biochemistry 5, page 467 et seq., (1966) and Analytical Biochemistry, 104, pages 300-310 (1980).

Specific examples of pH buffers within a pH range of 5.5 to 9.0 include buffers containing tris-(hydroxymethyl)aminomethane (Tris); buffers containing phosphates; buffers containing borates; buffers containing citric acid or citrates; buffers containing glysine; N,N-bis(2-hydroxyethyl)glycine (Bicine); a Na or K salt of N-2-hydroxyethylpiperazine-N′-2-hydroxypropane-3-sulfonic acid (HEPPS); a Na or K salt of N-2-hydroxyethylpiperazine-N′-3-sulfonic acid (EPPS); a Na or K salt of N-[tris(hydroxymethyl)methyl]-3-aminopropanesulfonic acid (TAPS); a Na or K salt of N-2-hydroxyethylpiperazine-N'-2-ethanesulfonic acid (HEPES); and acids, alkalis or salts to be combined with any of these above compounds as required. Specific examples of preferred buffers are potassium dihydrogen phosphate-disodium hydrogen phosphate; Tris-sodium borate; Tris-sodium borate-EDTA•2Na salt; Tris-citric acid; citric acid-sodium dihydrogen phosphate; Bicine; HEPPS; HEPPS sodium salt: EPPS; EPPS sodium salt; TAPS; TAPS sodium salt.

Preferably, the pH buffer is included in the layer containing an enzyme. The pH buffer is preferably included in the porous spreading layer or the reagent layer containing the enzyme. On the other hand, a low-molecular-weight pH buffer can move through layers with the penetration of water which is a medium for the applied aqueous liquid sample, and therefore does not have to be contained in all of the layers containing the enzyme and the color reagent composition. Such a low-molecular-wieght pH buffer may be included only in the hydrophilic layer or the water absorbing layer, for example.

Preferably, from the standpoint of production, packing, transportation, storage and analyzing operation, the multilayer analysis element of this invention is cut to small pieces in the shape of a square having one side measuring from 15 mm to 30 mm or in the shape of a circle having much the same size, and formed into analyzing slides by setting them into slide frames as disclosed, for example, in Japanese Patent Application (OPI) Nos. 63452/1982, and 156079/1979, Japanese Utility Model Application (OPI) Nos. 142454/1981 and 32350/1983 and Japanese Patent Application Publication No. 501144/1983 (based on PCT Application).

The analysis sample may be any aqueous sample containing cholesterol, and may include, for example, blood samples such as whole blood, plasma or serum samples, other various body fluids, foods, and process controlling samples for plants which produce or utilize cholesterol.

In accordance with the methods described in the above-cited patent documents, 5 $\mu\ell$ to 30 $\mu\ell$, preferably 8 $\mu\ell$ to 15 $\mu\ell$ of the aqueous liquid sample is spotted on the spreading layer of the multilayer analysis element, and as required incubated at a substantially constant temperature in the range of 20°C to 45°C, preferably 35 °C, to 40 °C,. Thereafter, the color formed in the analysis element is reflected and measured, for example, from the side of the light-transmissive support, and on the principle of colorimetry, the total cholesterol content of the liquid sample can be determined.

The following examples are given for illustrative porpuses only.

Example 1

On a polyethylene terephthalate (PET) film, 180 $\mu$m thick having a gelatin subbing layer, gelatin (26 g/m$^2$), nonyl phenoxy polyethoxy ethanol (containing an average number of oxyethylene units within the range of from 9 to 10; the number of oxyethylene units will be abbreviated hereinbelow as n) (0.03 g/m$^2$) and bis(vinylsulfonylmethyl) ether (30 mg/m$^2$) were coated and dried to form a gelatin layer (water-absorbing layer).

Water was supplied as moistening water to the water-abosrbing layer at a rate of 30 g/m$^2$, and a tricot knitted fabric (average thickness 250 $\mu$m) composed of spun yarns (50 denier) of polyethylene terephthalate (PET) rendered hydrophilic by glow discharge was laminated and bonded to form a spreading layer.

Then, an aqueous solution of a cholesterol analysis color reagent composition was coated on the knitted fabric spreading layer and dried so that the coating amounts of its components in the spreading layer were as shown below. Thus, a multilayer analysis element for total cholesterol determination was produced.

| The amounts of the components of the cholesterol analysis color reagent composition (per m$^2$) | |
|---|---|
| Methyl cellulose (methoxy group content 29%, viscosity 112 cps as a 2% by weight aqueous solution at 20°C) | 3.0 g |
| Fine particles of titanium dioxide (rutile type; particle size 0.25 - 0.40 μm) | 24 g |
| Octyl phenoxy polyethoxy ethanol (n = 40 on an average) | 1.1 g |
| Sodium deoxycholate | 1.5 g |
| Cholesterol oxidase (EC 1.1.3.6) | 3500 IU |
| Cholesterol esterase (EC 3.1.1.13) | 2000 IU |
| Peroxidase (EC 1.11.1.7) | 2900 IU |
| Sodium 2-hydroxy-3,5-bichlorobenzenesulfonate | 1.8 g |
| 4-Aminoantipyrine | 0.3 g |
| Sodium dihydrogen phosphate | 7.3 g |
| NaOH   to adjust the pH to 8.0 | |

Comparative Example 1

Example 1 was repeated except that octyl phenoxy polyethoxy ethanol (n = 40 on an average, nonyl phenoxy polyethoxy ethanol (n = within the range of 9-10 on an average) and sodium deoxycholate were changed in amounts or newly added as indicated in the Table below in 9 different cases. Thus, comparative multilayer analysis elements for total cholesterol determination were produced.

Each of the multilayer analysis elements produced in Example 1 and Comparative Example 1 was cut into a square chip having a size of 15 mm × 15 mm, and set in a plastic mount of the type disclosed in Japanese Patent Application (OPI) NO. 63452/1982 to form a chemical analysis slide for the determination of total cholesterol.

These slides were evaluated in the following manner.

Experiment-1

The above-described chemical analysis slide was stored for 3 days at 4°C at a relative humidity of not more than 5%, and at 45°C and a relative humidity of about 45%. Optical densities of the colors formed were compared. 10 μℓ of a human plasma sample having a cholestrol concentration of about 450 mg/dl taken by using heparin was spotted on each slide and incubated at 37°C for 6 minutes. The reflecting optical density was measured from the side of the PET support with light having a wavelength of 540 nm.

Experiment-2

The total cholesterol levels of human plasma samples having different total cholesterol levels were measured by the solution method of C. C. Allain et al. as a standard method [CLINICAL CHEMISTRY, 20, 470-475, (1974)]. On the basis of the results obtained, a calibration curve for human plasma was prepared for the multilayer analysis elements of the present invention and the comparisons.

Using human plasma having a neutral fat concentration of about 620 mg/dl, the same operation as in Experiment-1 was performed to form the colored product. The total cholesterol content was determined from the calibration curve.

Experiment-3

Using human plasma having a total protein content of about 11 g/dl, the same operation as in Experimet-1 was performed, and the colored product was formed. The total cholesterol content was determined from the above calibration curve.

The Table below summarizes the ratios of the total cholesterol content obtained by the chemical analysis slide to that obtained by the method of Allain et al. in human plasma samples in Experiment-2 and Experiment-3.

The results given in the Table below clearly show that (1) the multilayer analysis elements for total cholesterol determination in accordance with this invention have very small decrease (less than 1.3%) in the optical density of the colored product even after the accelerated deterioration test at 45°C and 45% RH for 3 days; (2) as in the solution method of C. C. Allain et al. as the standard measuring method, interference of the analysis due to neutral fats in the blood (about -1%) and total proteins in the blood (about -3%) is negligible with the elements of the present invention; and as overall properties, the element of the present invention have a very good correlation with the standard method.

Table

| Multilayer analysis element | Example 1 Invention | Comparative Example | | | | | | | | |
|---|---|---|---|---|---|---|---|---|---|---|
| | | 1-1 | 1-2 | 1-3 | 1-4 | 1-5 | 1-6 | 1-7 | 1-8 | 1-9 |
| OPhPEE n: average 9-10 | — | 7.0g | 15g | — | — | — | — | — | 7.0g | 15g |
| NPhPEE n:average 40 | 1.1g | — | — | — | 1.5g | 6.0g | — | — | — | — |
| Sodium deoxycholate | 1.5g | — | — | — | — | — | 1.5g | 6.0g | 4.0g | 4.0g |
| Experiment-1 (Reflection optical density values) | | | | | | | | | | |
| 4°C, not more than 5% RH | 0.852 | 0.791 | 0.760 | 0.610 | 0.720 | 0.740 | 0.772 | 0.792 | 0.780 | 0.760 |
| 45°C45%RH | 0.841 | 0.701 | 0.521 | 0.603 | 0.710 | 0.709 | 0.770 | 0.784 | 0.623 | 0.592 |
| Difference between 4°C and 45°C | 0.011 | 0.090 | 0.239 | 0.007 | 0.010 | 0.031 | 0.002 | 0.008 | 0.157 | 0.168 |
| Experiment-2 Ratio to the Allain method | 0.99 | 1.0 | 0.98 | 0.62 | 0.84 | 0.82 | 0.97 | 0.95 | 0.96 | 0.98 |
| Experiment-3 Ratio to the Allain method | 0.97 | 0.92 | 0.93 | 0.71 | 0.94 | 0.96 | 0.82 | 0.87 | 0.91 | 0.94 |

Note: OPhPEE: octyl phenoxy polyethoxy ethanol
NPhPEE: nonyl phenoxy polyethoxy ethanol

Patent documents written in English and corresponding to Japanese Patent documents referred hereinabove are listed below.

10

| | |
|---|---|
| JPA: Japanese Patent Application (OPI) No. | |
| JPP: Japanese Patent Publication No. | |
| UMP: Utility Model Application (OPI) No. | |
| JPAP: Japanese Patent Application Publication No. | |
| JPA No. 96378/78 | U.S. Patent 4,275,151, U.S. Patent 4,275,152 |
| JPA No. 85200/86 | U.S. Patent 4,680,259 |
| JPA No. 125796/75 | GB 1,479,994 |
| JPA No. 171864/84 | EP 0 119 861A |
| JPA No. 108753/85 | EP 0 142 849A |
| JPP No. 21677/78 | U.S. Patent 3,992,158 |
| JPA No. 90859/80 | U.S. Patent 4,258,001 |
| JPA No. 164356/80 | U.S. Patent 4,292,272 |
| JPA No. 66359/82 | GB 2 087 074A |
| JPA No. 222769/85 | EP 0 162 302A |
| JPA No. 145965/84 | EP 0 115 873A |
| JPP No. 45599/81 | U.S. Patent 3,983,005 |
| JPP No. 25840/80 | U.S. Patent 3,886,045 |
| JPA No. 54962/84 | EP 0 103 901A |
| JPP No. 18628/83 | U.S. Patent 4,042,335 |
| JPA No. 124398/81 | U.S. Patent 4,350,762 |
| JPA No. 155852/81 | U.S. Patent 4,291,121 |
| JPP No. 5519/82 | U.S. Patent 4,089.747 |
| JPA No. 193352/84 | EP 0 122 641A |
| JPA No. 4960/86 | EP 0 165 588A |
| JPA No. 156079/79 | U.S. Patent 4,169,751 |
| JUMP No. 142454/81 | U.S. 4.387.990 |
| JPAP No. 501144/83 | WO 83/00391 |

**Claims**

1. A multilayer analysis element for determination of total cholesterol comprising a light-transmissive water-impermeable support, at least one hydrophilic polymer layer on said support and a spreading layer on said hydrophilic polymer layer(s), and containing a cholesterol analysis color reagent composition comprising:
   (a) at least one enzyme having cholesterol estrase activity,
   (b) cholesterol oxidase,
   (c) peroxidase, and
   (d) a color reagent composition; wherein said element further contains:
   (e) at least one bile acid compound selected from the group consisting of bile acids, bile acid derivatives and salts of said acids and derivatives, and
   (f) an alkyl phenoxy polyethoxy ethanol containing a polyoxyethylene chain composed of at least 16 oxyethylene units.

2. The multilayer analysis element of claim 1, wherein the enzyme having cholesterol esterase activity is cholesterol esterase.

3. The multilayer analysis element of claim 1, wherein said bile acid compound is selected from the group consisting of cholic acid, deoxycholic acid, lithocholic acid, kenodeoxycholic acid, taurocholic acid, glycolic acid, taurodeoxycholic acid, glycodeoxycholic acid and sodium, potassium and lithium salts of these bile acids.

4. The multilayer analysis element of claim 1, wherein said alkylphenoxy polyethoxy ethanol is an alkyl phenoxy polyethoxy ethanol in which the alkyl group is linear or ranched alkyl group having 1 to 20

11

carbon atoms.

5. The multilayer analysis element of claim 1, wherein the number of oxyethylene groups in the polyoxyethylene chain in said alkyl phenoxy polyethoxy ethanol is from 16 to 60.

6. The multilayer analysis element of claim 1, wherein the number of said oxyethylene units in the alkyl phenoxy polyethoxy ethanol is from 20 to 50 on an average.

7. The multilayer analysis element of claim 1, wherein said color reagent composition comprises a chromogen and a coupler.

8. The multilayer analysis element of claim 1, wherein said color reagent composition comprises a leuco dye.

9. The multilayer analysis element of claim 1, wherein the element further contains a buffering agent.

10. The multilayer analysis element of claim 1, wherein the cholesterol analysis color reagent composition further contains a ferrocyanide compound.

11. The multilayer analysis element of claim 1, wherein the amount of said bile acid compound is 0.2 g to 10.0 g per $m^2$ of the element.

12. The multilayer analysis element of claim 1, wherein the amount of said alkyl phenoxy polyethoxy ethanol is 0.5 g to 12.0 g per $m^2$ of the element.

13. The multilayer analysis element of claim 1, wherein the amount of said enzyme having cholesterol esterase activity is 500 to 50,000 IU. (as the cholesterol esterase activity value) per $m^2$ of the element (the same hereinafter), the amount of said cholesterol oxidase is 1,000 to 30,000 IU, the amount of said peroxidase is 1,000 to 100,000 IU, the amount of a chromogen or leuco dye used in said color reagent composition is 0.5 to 10 millimoles, the amount of a coupler used in said color reagent composition is 2.5 to 50 millimoles.

14. The multilayer analysis element of claim 10, wherein the amount of said ferrocyanide is 0.1 to 10 millimoles per $m^2$ of the element.

15. The multilayer analysis element of claim 1, wherein the element includes a water-absorbing layer on the support and a layer containing the color reagent composition layer on said water-absorbing layer.

16. The multilayer analysis element of claim 1, wherein said enzyme having cholesterol esterase activity is contained in the spreading layer.

17. The multilayer analysis element of claim 1, wherein the bile acid compound is included in a layer the same or adjacent to the layer containing the alkyl phenoxy polyethoxy ethanol.

18. The multilayer analysis element of claim 1, wherein the number of said oxyethylene units in the alkyl phenoxy polyethoxy ethanol is from 30 to 50 on an average.

**Patentansprüche**

1. Mehrschichtiges analytisches Element zur Bestimmung von Gesamtcholesterin, dadurch **gekennzeichnet,** daß es einen lichtdurchlässigen, wasserundurchlässigen Träger, mindestens eine hydrophile Polymerschicht auf dem Träger und eine Ausbreitungsschicht auf der hydrophilen Polymerschicht bzw. den hydrophilen Polymerschichten enthält, und daß es eine Farbreagenz-Zusammensetzung zur Analyse von Cholesterin enthält, welche

    (a) mindestens ein Enzym mit CholesterinesteraseAktivität,
    (b) Cholesterinoxidase,
    (c) Peroxidase, und
    (d) eine Farbreagenz-Zusammensetzung enthält, wobei das Element weiterhin

12

EP 0 272 578 B1

(e) mindestens eine Gallensäureverbindung, ausgewählt aus der Gruppe bestehend aus Gallensäuren, Gallensäurederivaten und Salzen von Gallensäuren und -derivaten, und

(f) ein Alkylphenoxypolyethoxyethanol, enthaltend eine Polyoxyethylenkette mit mindestens 16 Oxyethyleneinheiten, enthält.

2. Mehrschichtiges analytisches Element nach Anspruch 1, dadurch **gekennzeichnet,** daß das Enzym mit Cholesterinesterase-Aktivität Cholesterinesterase ist.

3. Mehrschichtiges analytisches Element nach Anspruch 1, dadurch **gekennzeichnet,** daß die Gallensäureverbindung aus der Gruppe bestehend aus Cholsäure, Desoxycholsäure, Lithocholsäure, Chenodesoxycholsäure, Taurocholsäure, Glykolsäure, Taurodesoxycholsäure, Glykodesoxycholsäure und Natrium-, Kalium- und Lithiumsalzen von diesen Gallensäuren ausgewählt ist.

4. Mehrschichtiges analytisches Element nach Anspruch 1, dadurch **gekennzeichnet,** daß das Alkylphenoxypolyethoxyethanol ein Alkylphenoxypolyethoxyethanol ist, worin die Alkylgruppe eine lineare oder verzweigte Alkylgruppe mit 1 bis 20 Kohlenstoffatomen ist.

5. Mehrschichtiges analytisches Element nach Anspruch 1, dadurch **gekennzeichnet,** daß die Anzahl der Oxyethylengruppen in der Polyoxyethylenkette in dem Alkylphenoxypolyethoxyethanol16 bis 60 beträgt.

6. Mehrschichtiges analytisches Element nach Anspruch 1, dadurch **gekennzeichnet,** daß die Anzahl der Oxyethyleneinheiten in dem Alkylphenoxypolyethoxyethanol durchschnittlich 20 bis 50 beträgt.

7. Mehrschichtiges analytisches Element nach Anspruch 1, dadurch **gekennzeichnet,** daß die Farbreagenz-Zusammensetzung ein Chromogen und einen Kuppler enthält.

8. Mehrschichtiges analytisches Element nach Anspruch 1, dadurch **gekennzeichnet,** daß die Farbreagenz-Zusammensetzung einen Leukofarbstoff umfaßt.

9. Mehrschichtiges analytisches Element nach Anspruch 1, dadurch **gekennzeichnet,** daß das Element weiterhin ein Puffermittel enthält.

10. Mehrschichtiges analytisches Element nach Anspruch 1, dadurch **gekennzeichnet,** daß die Farbreagenz-Zusammensetzung zur Analyse auf Cholesterin weiterhin eine Ferrocyanidverbindung enthält.

11. Mehrschichtiges analytisches Element nach Anspruch 1, dadurch **gekennzeichnet,** daß die Menge der Gallensäureverbindung 0,2 g bis 10,0 g pro m$^2$ des Elements beträgt.

12. Mehrschichtiges analytisches Element nach Anspruch 1, dadurch **gekennzeichnet,** daß die Menge des Alkylphenoxypolyethoxyethanols 0,5 g bis 12,0 g pro m$^2$ des Elements beträgt.

13. Mehrschichtiges analytisches Element nach Anspruch 1, dadurch **gekennzeichnet,** daß die Menge des Enzyms mit Cholesterinesterase-Aktivität 500 bis 50.000 IU (als Wert für die Cholesterinesterase-Aktivität) pro m$^2$ des Elements (nachstehend dasselbe) beträgt, die Menge der Cholesterinoxidase 1.000 bis 30.000 IU beträgt, die Menge der Peroxidase 1.000 bis 100.000 IU beträgt, die Menge eines Chromogens oder Leukofarbstoffs, welche in der Farbreagenz-Zusammensetzung verwendet werden, 0,5 bis 10 mMol beträgt, die Menge eines Kupplers, der in der Farbreagenz-Zusammensetzung verwendet wird, 2,5 bis 50 mMol beträgt.

14. Mehrschichtiges analytisches Element nach Anspruch 10, dadurch **gekennzeichnet,** daß die Menge des Ferrocyanids 0,1 bis 10 mMol pro m$^2$ des Elements beträgt.

15. Mehrschichtiges analytisches Element nach Anspruch 1, dadurch **gekennzeichnet,** daß das Element eine wasserabsorbierende Schicht auf dem Träger und eine Schicht, die die Schicht mit der Farbreagenzzusammensetzung auf der wasserabsorbierenden Schicht enthält, enthält.

13

**16.** Mehrschichtiges analytisches Element nach Anspruch 1, dadurch **gekennzeichnet,** daß das Enzym mit Cholesterinesterase-Aktivität in der Ausbreitungsschicht enthalten ist.

**17.** Mehrschichtiges analytisches Element nach Anspruch 1, dadurch **gekennzeichnet,** daß die Gallensäureverbindung in einer Schicht, die mit der Schicht, die das Alkylphenoxypolyethoxyethanol enthält, identisch ist oder dieser Schicht benachbart ist, enthalten ist.

**18.** Mehrschichtiges analytisches Element nach Anspruch 1, dadurch **gekennzeichnet,** daß die Anzahl der Oxyethyleneinheiten in dem Alkylphenoxypolyethoxyethanol durchschnittlich 30 bis 50 beträgt.

**Revendications**

**1.** Elément d'analyse à couches multiples pour la détermination du cholestérol total, comprenant un support transparent imperméable à l'eau, au moins une couche polymère hydrophile sur ledit support et une couche de diffusion sur la ou lesdites couches polymères hydrophiles, et contenant une composition de réactif colorant pour l'analyse du cholestérol, comprenant
    (a) au moins une enzyme ayant une activité de cholestérol-estérase,
    (b) la cholestérol-oxydase,
    (c) une peroxydase, et
    (d) une composition de réactif colorant ledit élément contenant en outre
    (e) au moins un composé acide biliaire choisi dans le groupe formé d'acides bilaires, de dérivés d'acides biliaires et de sels desdits acides et dérivés, et
    (f) un alcoyl-phénoxy-polyéthoxy-éthanol contenant une chaîne de polyoxyéthylène composée d'au moins 16 unités oxyéthylène.

**2.** Elément d'analyse à couches multiples selon la revendication 1, dans lequel l'enzyme ayant une activité de cholestérol-estérase est la cholestérol-estérase.

**3.** Elément d'analyse à couches multiples selon la revendication 1, dans lequel le composé acide biliaire est choisi dans le groupe formé de l'acide cholique, l'acide désoxycholique, l'acide lithocholique, l'acide chénodésoxycholique, l'acide taurocholique, l'acide glycolique, l'acide laurodésoxycholique, l'acide glycodésoxycholique et les sels de sodium, de potassium et de lithium de ces acides biliaires.

**4.** Elément d'analyse à couches multiples selon la revendication 1, dans lequel ledit alcoylphénoxy-polyéthoxy-éthanol est un alcoyl-phénoxy-polyéthoxy-éthanol dans lequel le groupe alcoyle est un groupe alcoyle linéaire ou ramifié ayant de 1 à 20 atomes de carbone.

**5.** Elément d'analyse à couches multiples selon la revendication 1, dans lequel le nombre de groupes oxyéthylène dans la chaîne de polyoxyéthylène dudit alcoyl-phénoxy-polyéthoxy-éthanolvaut de 16 à 60.

**6.** Elément d'analyse à couches multiples selon la revendication 1, dans lequel le nombre desdites unités oxyéthylène de l'alcoyl-phénoxy-polyéthoxy-éthanol vaut de 20 à 50 en moyenne.

**7.** Elément d'analyse à couches multiples selon la revendication 1, dans lequel ladite composition de réactif colorant comprend un agent chromogène et un copulant.

**8.** Elément d'analyse à couches multiples selon la revendication 1, dans lequel ladite composition de réactif colorant comprend un leuco-colorant.

**9.** Elément d'analyse à couches multiples selon la revendication 1, contenant en outre un agent tampon.

**10.** Elément d'analyse à couches multiples selon la revendication 1, dans lequel la composition de réactif colorant pour l'analyse du cholestérol contient en outre un composé ferrocyanure.

**11.** Elément d'analyse à couches multiples selon la revendication 1, dans lequel la quantité dudit composé acide biliaire est de 0,2 g à 10,0 g par m$^2$ d'élément.

**12.** Elément d'analyse à couches multiples selon la revendication 1, dans lequel la quantité dudit alcoyl-phénoxy-polyéthoxy-éthanol est de 0,5 g à 12,0 g par m$^2$ d'élément.

**13.** Elément d'analyse à couches multiples selon la revendication 1, dans lequel la quantité de ladite enzyme ayant une activité de cholestérol-estérase est de 500 à 50 000 U.I. (comme valeur d'activité de cholestérol-estérase) par m$^2$ d'élément, la quantité de ladite cholestérol-oxydase est de 1 000 à 30 000 U.I. par m$^2$ d'élément, la quantité de ladite peroxydase est de 1 000 à 100 000 U.I. par m$^2$ d'élément, la quantité d'agent chromogène ou de leuco-colorant utilisé dans ladite composition de réactif colorant est de 0,5 à 10 millimoles par m$^2$ d'élément et la quantité de copulant utilisé dans ladite composition de réactif colorant est de 2,5 à 50 millimoles par m$^2$ d'élément.

**14.** Elément d'analyse à couches multiples selon la revendication 10, dans lequel la quantité dudit ferrocyanure est de 0,1 à 10 millimoles par m$^2$ d'élément.

**15.** Elément d'analyse à couches multiples selon la revendication 1, comprenant une couche hydrophile sur le support et une couche contenant la couche de composition de réactif colorant sur ladite couche hydrophile.

**16.** Elément d'analyse à couches multiples selon la revendication 1, dans lequel ladite enzyme ayant une activité de cholestérol-estérase est contenue dans la couche de diffusion.

**17.** Elément d'analyse à couches multiples selon la revendication 1, dans lequel le composé acide biliaire est contenu dans une couche qui est la même ou adjacente à la couche contenant l'alcoyl-phénoxy-polyéthoxy-éthanol.

**18.** Elément d'analyse à couches multiples selon la revendication 1, dans lequel le nombre d'unités oxyéthylène de l'alcoyl-phénoxy-polyéthoxy-éthanol vaut de 30 à 50 en moyenne.